Europäisohes Patentamt

European Patent Office

Office européen des brevets

(19)

(11) Publication number: **0 451 686 A2**

# EUROPEAN PATENT APPLICATION

(21) Application number: 91105283.5

(22) Date of filing: 03.04.91

(51) Int. Cl.5: **G01N 33/543, //G01N33/569**

(30) Priority: **12.04.90 US 509561**

(43) Date of publication of application:
**16.10.91 Bulletin 91/42**

(84) Designated Contracting States:
**DE FR GB IT**

(71) Applicant: **MILES INC.**
**1127 Myrtle Street**
**Elkhart Indiana 46514(US)**

(72) Inventor: **Miller, Carol A.**
**51101 Winding Water Lane N.**
**Elkhart, IN 46514(US)**
Inventor: **Sharma, Harmesh K.**
**17420 Parker**
**South Bend, IN 46635(US)**

(74) Representative: **Dänner, Klaus, Dr. et al**
**c/o Bayer AG Konzernverwaltung RP**
**Patentabteilung**
**W-5090 Leverkusen 1 Bayerwerk(DE)**

(54) Immunoassay test device with thread control element.

(57) A test device for use in immunoassay methods wherein a liquid test mixture is passed through a porous solid support and wherein analyte that becomes immobilized at the surface of said solid support is determined by reaction with a reagent system to produce an optical response, e.g., the appearance of color, on said surface. The test device comprises a thread in liquid flow contact with and extending across the test surface; the thread being incorporated with a control reagent that reacts with one or more components of the reagent system to produce an optical response on the thread independent of the presence of the analyte in the test medium.

EP 0 451 686 A2

## BACKGROUND OF THE INVENTION

The present invention relates to immunoassay test devices which comprise a control reagent for assessing the function of assay reagent components during the course of performing an assay. More particularly, the invention concerns the application of control reagents in flow-through immunoassay test devices.

Over the past several years, a number of different test devices have been developed for use in performing rapid and convenient immunoassays. Such devices have in particular been successful in enabling immunoassay testing to be performed by relatively untrained operators such as patients, or prospective patients, themselves and physician office personnel. Rapid immunoassay tests are currently sold for detection of hCG (pregnancy), LH (ovulation), and Streptococcus A (strep throat).

Flow-through test devices are the most commonly used devices of this type today. Such devices have a porous solid support such as a filter or membrane which has a test surface which is capable of ultimately immobilizing an immune complex comprising the analyte to be detected bound with a labeled antibody reagent. Usually, the filter or membrane is held in contact with an absorbent material which serves to draw liquid through the test surface. After a liquid test medium has been applied to and drawn through the test surface, one or more further liquid reagents are applied to cause the formation of color or other optical signal on the test surface if sufficient analyte is present in the liquid medium tested.

Because these flow-through immunoassay devices are intended for use by relatively untrained individuals, there is a recognized need for incorporating controls which signal to the users that they have correctly performed the test and that the reagents are functioning properly. Improper technique, whether in the application or processing of a test sample or in the stepwise performance of the test, can produce a false negative result, i.e., no observable signal is observed for a test sample which in fact is positive for the analyte. Similarly, a false negative result can be obtained if the reagents have deteriorated such as due to improper storage or handling.

A number of approaches have been conceived for incorporating controls which to a varying degree will indicate to the user whether he or she has performed the test correctly and that the reagents are functioning properly. The most common approach has been to immobilize an appropriate control reagent on the test surface itself such as by applying to the test surface a suspension of particles carrying the control reagent. The particles are selected to become entrapped in the support material and usually are applied in a pattern which distinguishes the control response from a positive test result, e.g., the "plus-minus", or "+/-", patterns found in today's commercial devices.

Representative flow-through immunoassay devices, including some with a control feature, are described in U.S. Pat. Nos. 4,623,461; 4,632,901; 4,693,834; 4,727,019; and 4,818,677; in European Published Patent Applications 186,100; 200,381; 217,403; 249,418; 253,464; and 269,876; and in PCT Publication 87-03690.

The prior art methods for incorporating control reagents with flow-through immunoassay test devices have significant disadvantages. They generally require the use of variable synthetic methods for coupling or fixing the desired control reagents to particles. Further, complex and sensitive equipment is needed to control the application of reagent particles to the test surface, while the end product nonetheless often suffers from poor definition of the control reagent area on the test surface.

## SUMMARY OF THE INVENTION

The present invention provides a flow-through immunoassay test device in which a control reagent is incorporated with a thread element extending across the surface of a test surface. The test device is used in methods in which a liquid test medium, e.g., a test sample or a liquid mixture that comprises a diluted or processed test sample, is passed through a porous solid support and analyte that becomes immobilized at the surface of the solid support is determined by reaction with a reagent system to produce an optical response, such as the appearance of color, on the test surface. As described in more detail hereinafter, a variety of immunoassay test formats can be performed using such a flow-through device for separation and detection. In general, the test surface of the solid support is porous to the liquid test medium and is prepared to be capable of immobilizing analyte or analyte that is complexed or bound by a specific binding partner thereof, e.g., an antibody. The present thread element is placed in liquid flow contact with and extending across such test surface and is incorporated with a control reagent that reacts with one or more components of the reagent system to produce an optical response on the thread independent of the presence of analyte in the test medium.

The thread control can provide a primary, secondary, or tertiary control. As a primary control, the incorporated control reagent will be the analyte or an analog thereof and thus will provide the optical test response only if all components of the reagent system are functioning properly. As a secondary or tertiary control, the incorporated control reagent will be a substance that reacts with less than all of the components of the reagent system.

The present invention is particularly useful in flow-through immunoassay test devices which provide what are commonly referred to as the "plus-minus", or "+/-", test responses. In such devices, a positive test result is indicated by the appearance of a "+" pattern of optical response whereas only a "-" pattern appears if the test is negative for analyte. The control thread element of the present invention can serve as the basis for the "-" pattern which indicates that the test reagent(s) are functioning properly when the test result is truly negative for analyte.

The use of a thread control element in accordance with the present invention provides a number of important advantages. Because of its tight linear structure, the thread control element provides a clear, sharply contrasting control response against the test surface background. In the case of color responses, this results in an enhanced ability to visually observe the control response. In addition, since the thread control elements is entirely exposed on the test surface, the entirety of the final optical response, e.g., color, is made available for observation. Further, test devices incorporating a thread control are much more readily manufacturable than the prior art devices which typically involve the patterned application of liquid dispersions of control reagent particles to the test surface. In contrast with such prior art devices which require careful volume, position, and environmental control in application of the control reagent, control threads of the present invention can be prepared in bulk and simply drawn across and fixed in position to the the test surface. Also, useful thread materials are easily procurable and the incorporation of control reagent with a thread element involves comparatively simple manufacturing operations.

BRIEF DESCRIPTION OF THE DRAWINGS

Fig. 1 is an isometric projection of a test device useful in performing an assay for Chlamydia trachomatis and incorporating a thread control element of the present invention.

Fig. 2 is an exploded diametric projection of the test device shown in Fig. 1.

Fig. 3 is a cross-sectional view of the device shown in Fig. 1.

Figs. 4 and 5 are top plan views of the Fig. 1 device, with Fig. 5 showing the device with its funneling element removed.

Figs. 6, 6a, and 6b show different test responses that are produced using the device of Fig. 1 in a Chlamydia assay method.

DESCRIPTION OF THE PREFERRED EMBODIMENTS

The two principle components of the test device of the present invention are (1) the porous solid support which comprises the test surface on which the optical test response is observed or measured, and (2) the control thread. Considering the porous test support first, the important characteristics of this element are that it be essentially porous to the test medium and liquid reagents that are to be applied in the course of performing a particular immunoassay method. Further, the test support will be capable of immobilizing either analyte or analyte bound by a specific binding partner, such as an antibody. This immobilization can result in an number of different ways to be described in more detail below. At this point it suffices to say that the test support serves to selectively separate analyte from the test medium and to make it available for detection at the test surface. Such detection occurs as the result of reaction with a reagent system that produces an optical response on the test surface, normally the appearance of color which can be readily observed visually or read with a suitable instrument.

The thread control element lays or is fixed in liquid flow contact with and extending across the exposed test surface of the porous test support. Such liquid flow contact intends that when liquid is in contact or passed through the test support, it also is or comes into contact with the thread element. Normally, the control thread will be held in actual physical contact with the test surface. The control thread is incorporated with a control reagent that reacts with one or more (depending upon whether the control is a primary, secondary, or other level control) components of the detectant reagent system to produce an optical response, normally the same type of response as the response of the detection system, independent of the presence or amount of analyte in the applied test medium.

Preferably, the test procedure and/or the test support are designed in a manner that the optical response of the detection reagent system (the test response) is in the form of a visual pattern on the test surface, for example, a small filled circle or dot or other geometric shape, or a line or bar ("-"). Also, the optical response on the control thread is preferably of the same nature as the test response, e.g., the same color makes its appearance in both cases. Thus, the resulting effect is that the test response and the control response are distinguishable by the patterns produced on the test surface. Even more preferable is the design of test and control patterns with cooperate to produce a unique pattern only when both the test and control responses are generated (for example, a plus sign or

" + "), thus signaling a "true" positive response. The optical responses of the detection and control reactions can be selected according to the needs and desires of the user. Color changes or the production or disappearance of color will generally be preferred. Fluorescent, chemiluminescent, or other optical responses can also be used.

A typical test device incorporated with the thread control element of the present invention will be particularly designed for performance of the test by relatively untrained individuals. For example, the test support can be housed in a small free-standing plastic device which exposes a suitable circular or other shaped test surface. Usually, the device will also house an absorbent reservoir enclosed below but in liquid flow contact with the test support in order to facilitate flow and containment of liquids after they have performed their functions in passing through the test support. Such devices are currently well known (see, for example, U.S. Patent Nos. 4,623,461; 4,632,901; 4,693,834; 4,727,019; and 4,818,677).

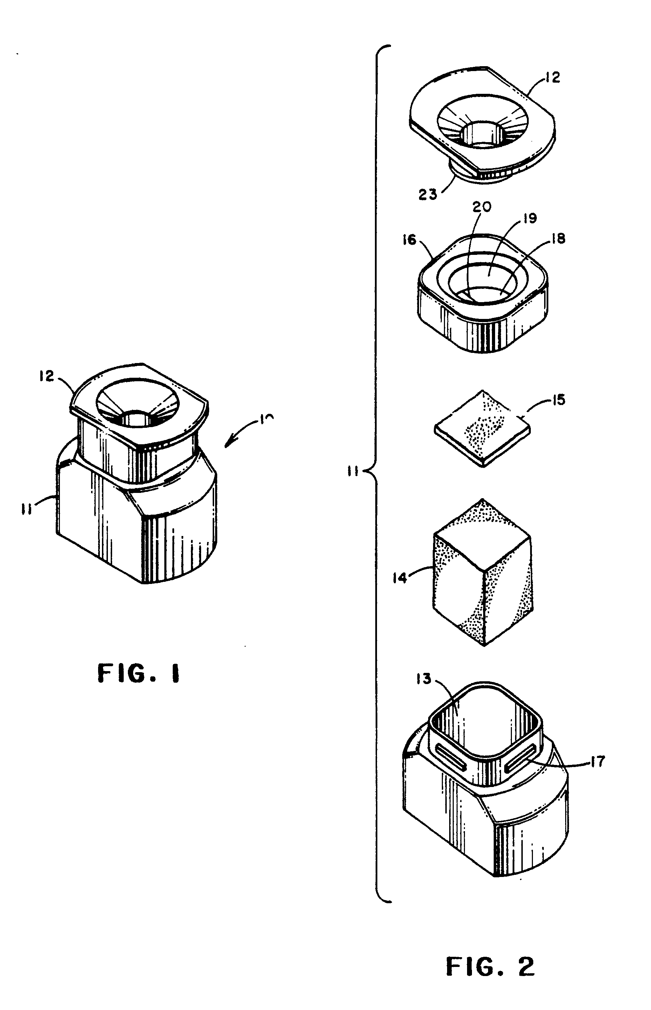

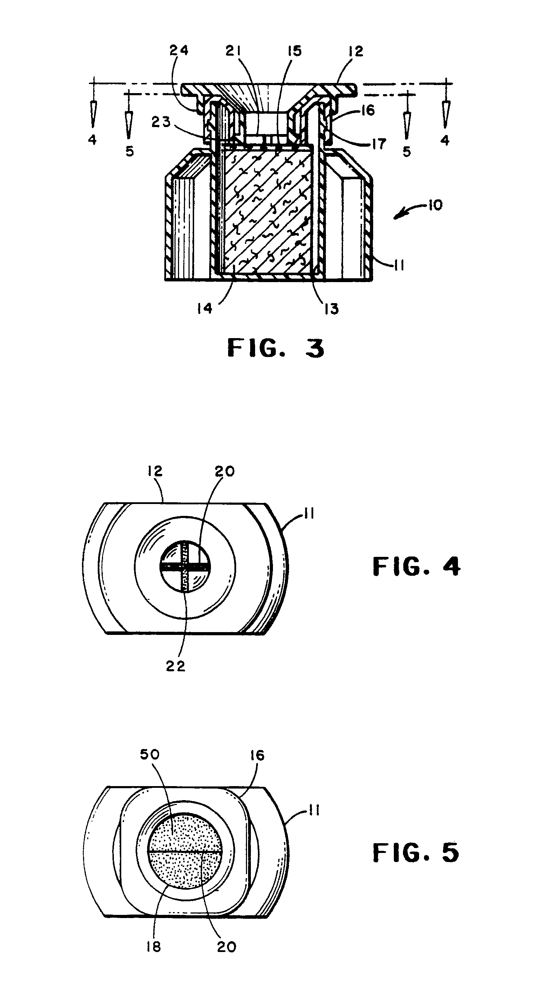

A particularly unique device that can be applied to the present invention is illustrated in the drawings. As illustrated in Figs. 1-3, test device 10 comprises a base member 11 and a removable funneling element 12. Base member 11 has a cavity 13 which accommodates both absorbent block 14 (formed of a highly water absorbent material such as Transorb® reservoirs available from American Filtrona Co., Richmond, VA, USA), and glass fiber filter 15 (e.g., cut from PD-008 12B 100 available from Whatman Paper Ltd., Kent, UK). Cap 16 is molded with an inner groove to snap fit onto ridges 17 on base member 11 and has a circular aperture 18 defined by the bottom edge of wall 19. The combined height of the absorbent block 14 and the glass fiber filter 15 is sufficient that they are held firmly in place by the bottom edge of wall 19 in the snap fit cap 16. Attached to the bottom edge of wall 19 in cap 16 and extending diametrically across aperture 18 is thread element 20 which is incorporated with a control reagent. As a result, when cap 16 is snap fit onto base member 11, thread control element 20 is held in physical contact with the surface of glass fiber filter 15 exposed in cap aperture 18. Fig. 5 is a top plan view of device 10 with funneling element 12 removed. Thread control element 20 is shown extending across surface 50 of glass fiber filter 15 exposed by cap aperture 18.

With reference to Figs. 3 and 4, bottom plate 21 of funneling element 12 has an aperture 22 which is shaped as a cross or plus sign (" + "). Further, funneling element 12 has an annular flange 23 and pegs 24 to hold same firmly in place when inserted into cap 16. Pegs 24 also serve to orient the funneling element when fit into cap 16 so that one of the cross bars of cross-shaped aperture 22 is coincident with control thread 20 extending across the surface of glass fiber filter 15 exposed in cap aperture 18.

An illustrated preferred use of test device 10 in an assay for the detection of Chlamydia is as follows. Separate from device 10, a test specimen such as a cervical or urethral swab is immersed in a specimen processing liquid which releases and exposes Chlamydia antigens, if any, present in the specimen. The swab is removed, leaving a liquid mixture which, in the case of a specimen that is positive for Chlamydia, contains multivalent Chlamydia antigens. Enzyme-labeled antibody reagent is added to the liquid mixture and incubated for a suitable period, e.g., 20 minutes, to allow formation of immune complexes with the Chlamydia antigens. The liquid test mixture is then poured, optionally after being pre-filtered, into funneling element 12 in assembled test device 10 thereby flowing through cross-shaped aperture 22, in contact with control thread 20, through glass fiber filter 15, and into absorbent block 14.

Labeled immune complexes are consequently retained by glass fiber filter 15 in a cross-shaped pattern on the exposed surface of such filter 15. Next, a wash solution is poured into the test device to wash the exposed surface of filter 15 and carry unbound labeled antibody and potentially interfering substances into absorbent block 14. Finally, an appropriate substrate/chromogen indicator solution is poured into the device resulting in the formation of color on the thread control element (if the test procedure has been followed correctly and the reagents are functional) and, in the case of a positive specimen, on the exposed filter surface in the pattern of a cross or plus sign.

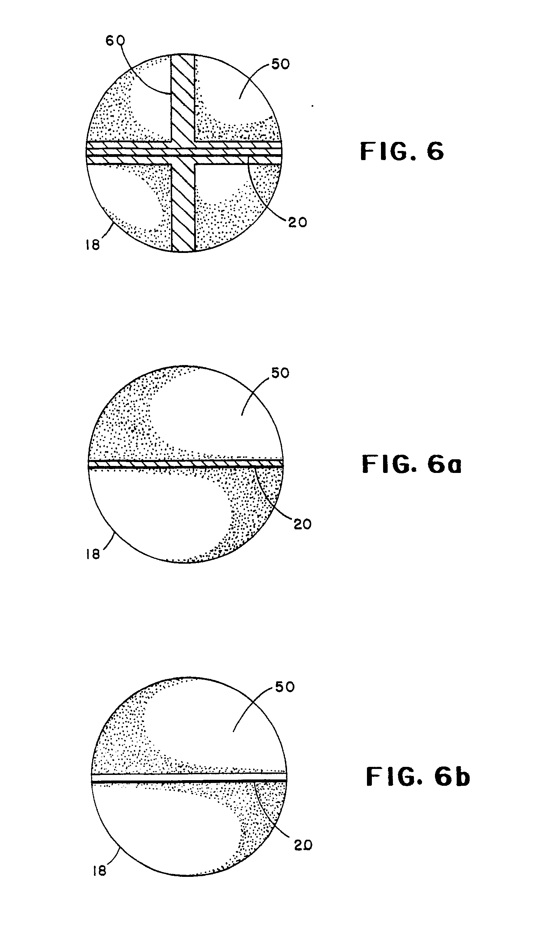

Figs. 6, 6a, and 6b illustrate possible test results appearing on the exposed surface 50 appearing within cap aperture 18. Fig. 6 illustrates the condition of the exposed test surface upon the conclusion of an assay on a specimen positive for Chlamydia. The cross-shaped area 60 exposed on the test surface by the funneling element, as well as control thread 20, has developed color (indicated by cross-hatching). Fig. 6a illustrates the condition of the exposed test surface upon the conclusion of an assay on a truly false specimen. No colored response appears on the exposed surface of the test surface, however, control thread 20 has turned color to indicate that the test procedure was correctly followed and the reagents were functional. Finally, Fig. 6b illustrates the condition of the exposed test surface upon the conclusion of an assay that was run incorrectly or with a nonfunctioning reagent. No colored pattern appears either on the exposed surface of the filter or on the control thread element indicating a false negative result. Such a test result calls for a repeat of the assay.

In general, the thread control element of the present invention will comprise an extended filamentous structure formed of a monofilament material or an associated bundle, e.g., twisted number, of filaments. The thread material selected can vary according to the particular needs of the test device to be constructed. Threads can be advantageously selected for flexibility as well as tensile strength, and can have varying chemical properties for purposes of incorporation of the control reagent. Natural and synthetic threads can be used and comprise numerous chemically reactive functional groups useful for hydrophobic, electrostatic, and covalent attachment of the control reagent to the thread. Useful threads will generally be inexpensive and commercially available. Some examples of thread materials which can be used in the present invention are 100% cotton (cellulose) available from a variety of sources such as J. & P. Coats, Charlotte, North Carolina, USA, and Wm. E. Wright Co., West Warren, MA, USA (Molnlyke); polyester, nylon, flax, and the like. Threads can be used in a variety of sizes, e.g., quilt weight, #8, #40, and #60 sizes just to name a few for illustrative purposes. In some case, threads of thicker diameters can produce non-homogeneous control responses along their surfaces, and thus, the thinner threads, e.g., #40, can be preferable.

Incorporation of a selected thread element with the control reagent can be accomplished in any desired manner which produces essential insolubilization, fixation, or immobilization of such reagent in or on the thread material. Usually, such is accomplished by noncovalent, e.g., hydrophobic or electrostatic, means, or by covalent coupling means. Considering that the control reagent can vary widely according to the particular detection system involved and depending upon whether a primary, secondary, or other level of control is desired, the availability of such diverse means of incorporation provides advantageous flexibility in obtaining maximum reactivity and stability of the incorporated reagent. For example, it has been found that low molecular weight peptides have limited ability to bind with detection system antibodies when incorporated by hydrophobic interactions, while covalent coupling to the thread maintains essentially full binding activity. Furthermore, in some instances, electrostatic interactions can provide higher loading of the control reagent on the thread compared to hydrophobic and covalent interactions.

Commercially available thread can be expected to have been chemically treated or incorporated with one or more unknown ingredients (e.g., mercerized to impart luster, strength, and dye receptivity). Therefore, in many cases it will be desired or necessary to pre-treat the thread in preparation of incorporating the control reagent. In the case of cotton threads, a typical pre-treatment can comprise immersion in detergent solution, base (e.g., 1.0 N sodium hydroxide) to disrupt crystalline structure and remove traces of nitrocellulose, acid (e.g., 1.0 N hydrochloric acid) to extract acid-soluble materials, and/or any number of organic solvents such as dimethylsulfoxide (DMSO), alcohol (e.g., methanol), or acetone to extract organic-soluble materials. Treated thread can be stored under desiccation. Other treatments for purposes of improving the quality of control reagent incorporation will be evident to the ordinary skilled person in the field.

Selection of a method for incorporating the control reagent with the thread element will essentially depend upon the native chemical properties of both materials or the ability to modify such properties. For instance, hydrophobic interaction can be relied upon for incorporation where the control reagent comprises significant hydrophobic groups, e.g., proteins such as antibodies. Furthermore, the thread can be modified to enhance hydrophobic interactions. In such cases, incorporation can be carried out by exposing the thread to the control reagent under conditions of high salt concentration to dissociate charge interactions and favor hydrophobic binding. As a specific example, hydrophobic attachment of protein control reagents has been accomplished by incubation at room temperature overnight at pH 9.5 in the presence of high salt buffers (normally having an ionic concentration of greater than about 0.2 M) at a protein:thread ratio of 1:50 (w/w). Weakly bound protein was removed by washing with guanidinium chloride at pH 7.0-7.3, followed by high pH and then low pH washes, with a final wash with phosphate buffered saline (PBS).

Incorporation by electrostatic interactions can similarly be applied in the case of control reagents which inherently comprise positively or negatively charged chemical groups or which have been chemically modified to introduce such groups. Conventional thread materials will normally require the introduction of charged chemical groups in order to obtain electrostatic interaction. As a specific example, electrostatic attachment of protein control reagents has been achieved by nitrating thread (using an aqueous solution of nitric and sulfuric acids, followed by reflux with 95% ethanol three times to remove potentially explosive nitro groups). The protein was then incubated with the thread at low salt concentration at neutral pH at a protein:thread ratio of 1:17 (w/w). Free binding sites were then blocked by overnight incubation with bovine serum albumin (BSA). Weakly bound material was removed by repeated washings with low salt buffer.

Covalent coupling of the control reagent involves the formation of a covalent bond between native or chemically introduced groups in both the thread and the particular control reagent involved. It is apparent that an extremely wide variety of coupling methods can be applied to this task. As merely a few specific examples, oxirane and Schiff's base coupling with borohydride reduction are mentioned here. In oxirane coupling, 0.2 mL of butandiol diglycidyl ether has been very successfully used with 100 mg of thread and 1.78 mg of protein control reagent. The reaction time has been about 24 hours at room temperature with washing using the conditions described above regarding hydrophobic attachment. The Schiff base method involves periodate oxidation of the thread to generate carbonyl groups to which a protein control reagent can be coupled through amino groups, forming a Schiff's base. The labile Schiff's base is stabilized by conventional borohydride reduction.

As introduced above, the control reagent can be selected to provide a primary, secondary, or tertiary control. A primary control is one which must react with all of the detection reagents in order to provide a response. Thus, a primary control indicates whether all detection reagents have been added in proper sequence and are all functioning properly. For example, a typical analyte detection system comprises an enzyme-labeled anti-analyte antibody and a substrate/chromogen indicator. A primary control reagent can be the analyte itself or an analog of the analyte which binds sufficiently with the enzyme-labeled antibody conjugate. Thus, if the labeled antibody conjugate and substrate/chromogen are added to the test device in proper order and each of the antibody in the conjugate, the enzyme label, and the substrate/chromogen indicator is functioning properly, i.e., have not deteriorated, then reaction with the control reagent incorporated with the thread will generate the optical response in and/or on the thread.

A secondary or tertiary control is one which can react with less than all of the detection reagents to provide the control response. Using the enzyme-labeled antibody example, a secondary control might test the functioning of the enzyme label and substrate/chromogen indicator but not the functioning of the antibody in the conjugate. Such a control reagent would be an antibody or antibody fragment directed against the labeled conjugate, e.g., an anti-antibody, a non-inhibiting anti-enzyme antibody, antibody directed against the linking group in the labeled conjugate, or the like as are well known in the art. A tertiary control would test the functioning of even fewer of the detection system components. For example, the enzyme used for labeling could be used as a tertiary control reagent and would indicate that the substrate/chromogen indicator was working properly.

The present invention applies to any immunoassay test device in which analyte ultimately becomes immobilized, and is detected by a reaction, at the surface of the solid test support. For example, the test surface can comprise an immobilized form of an antibody reagent directed against the analyte. Passage of a test sample containing analyte through the device results in binding and immobilization of analyte at the test surface by binding to the immobilized anti-analyte antibody. Preferably, the antibody reagent is immobilized on a limited area of the exposed test surface and in a pattern that, cooperatively with the thread control element extending across the test surface, provides a pattern of optical response indicative of a positive test result when the test sample contains a detectable amount of analyte. Such a pattern is provided by immobilizing the anti-analyte antibody on the test surface as a thin band positioned perpendicular to the thread whereby a positive test result produces a " + " pattern of response while a true negative test result produces only a "-" pattern of response due to production of response only on the thread.

An essentially similar effect can be obtained by immobilizing anti-analyte antibody across substantially the entire exposed test surface but applying the test sample to only an area limited to a thin band along the length of the control thread and a crossing thin band oriented perpendicular to the thread. Such sample application can be accomplished using a funneling element which is either a removable part of the test device or comprises the outlet of a sample applicator.

Alternatively to immobilizing anti-analyte antibody on the test surface in order to immobilize analyte for detection, the test surface can be selected to be impervious to immune complexes formed between the analyte and an antibody reagent directed against the analyte. Such immune complexes can be otherwise soluble or suspendable in the test mixture, or can comprise latex or other particles as an aid to separation such as by using antibody-latex particles as a reagent. After incubating the test sample with the antibody reagent, preferably a labeled conjugate such as an enzyme-labeled antibody conjugate, passage of the test mixture through the device results in immobilization of analyte/antibody complexes at the test surface. Preferably, the test mixture is applied to a limited area of the exposed test surface and in a pattern that, as above, cooperatively with the thread control element extending across the test surface, provides a pattern of optical response indicative of a positive test result when the test sample contains a detectable amount of analyte. Such a pattern is provided by applying the test mixture to only an area limited to a cross pattern comprising a thin band extending along the length of the control thread and a crossing thin band oriented perpendicular to the thread. Such application of the test mixture can be accomplished using a funneling element which is either a removable part of the test device or comprises the outlet of a sample applicator. As above, a positive test result produces a " + " pattern of response while a true negative test result produces only a "-" pattern of response due to production of response only on the thread.

The present invention will now be illustrated, but is not intended to be limited by, the following examples.

EXAMPLE 1

Preparation of Primary Control Threads

Primary control threads have been prepared using both covalent and hydrophobic approaches to bind control antigen to the cotton thread. Using exhaustively cleaned cotton thread (described above), the thread and control reagent were stirred overnight (1.76 mg antigen:100 mg thread ratio) in 2.0 mL carbonate buffer (0.5 M, pH 9.5). Test antigens have included species-specific peptides (12-mer and 20-mer) of the chlamydia outer membrane protein complex previously covalently bound to keyhole limpet hemocyanin (KLH) or bovine serum albumin (BSA); ratios of antigen and carrier protein were 100:1, respectively. UV-irradiated Chlamydia trachomatis elementary bodies (EB) were attached to the thread by the same protocol except that the EB protein:thread ratio was 1.12:100 (w/w). After adsorption, the free ligands were removed by washing with 5 M guanidine hydrochloride (1 mL for 1-2 hr); followed by sequential washing in phosphate buffered saline, PBS (0.1 M $PO_4$, 0.15 M NaCl, pH 7.4, 3 X 2.0 mL), carbonate buffer (0.5 M carbonate, pH 9.5, 2.0 mL), and glycine buffer (0.1 M, pH 3.0, 2.0 mL). The thread was then washed with PBS (3 X 2.0 mL) and demonstrated to be reactive, i.e., segments laid atop a filter subsequently reacted with a conjugate, washed, and exposed to substrate, exhibited a blue color throughout the thread segment. As well, segments which had been exposed to the conjugate, washed, and then exposed to soluble substrate exhibited the formation of a soluble product which can be measured spectrophotometrically.

Primary control threads made with free peptide by the procedure described in the foregoing, i.e., not covalently linked to a carrier, were found to be unreactive when hydrophobically bound to the thread matrix. Alternatively, peptides covalently bound to the cotton thread, via hydrazone linkage (Schiff 's base chemistry) retained antibody binding capacity. For covalent binding, the washed thread (50 mg) was oxidized with 2.0 mL of 50 mM sodium metaperiodate in 10 mM acetate buffer, pH 4.0 at room temperature. After overnight reaction, the thread was made free of periodate by repeated washings with the acetate buffer. The peptide ligand (referred to as 20-mer), 1.16 mg, was incubated with 25-30 mg of the activated thread in 3 mL of 10 mM $CO_3/HCO_3$ buffer with 2.5 mM EDTA, pH 9.5. After incubation at $0°C$ for 24-48 hours, the reaction mixture was pipetted out and the free carbonyl groups were reduced with 1-1.2 mL of 1 M $NaBH_4$ in the carbonate buffer for 4 hours at $0°C$. The thread was then washed with PBS as described above.

Compared to the use of a hydrophobic interaction, electrostatic adsorption of the IgG has been shown to be a preferred approach, e.g., using either cotton or polyester thread as the starting matrix. This approach has the advantage of increasing the ligand load onto the thread using a reduced ligand:thread ratio. Briefly, in the case of a polyester thread, the thread is exposed to 40% $HNO_3$, 40% $H_2SO_4$, 20% $H_2O$ for a 60 minute nitration period at $15°C$ and subsequently reacted with rabbit IgG anti-goat IgG. In the case of a cotton thread, the thread is exposed to 64% $H_2SO_4$, 20% $HNO_3$, 16% $H_2O$ for a 10-15 minute incubation period at $15°C$ and then reacted with antibody. The thread control retained tensile strength and exhibited a strong reaction upon subsequent contact with the conjugate/substrate reagents. Stronger nitration conditions and longer nitration times can increase brittleness of thread and, therefore, would be avoided.

EXAMPLE 2

Preparation of Secondary Control Threads

Both cotton and polyester threads have been employed to prepare secondary control thread by hydrophobic and electrostatic binding of rabbit IgG anti-goat IgG to the thread matrix. Hydrophobic binding of the IgG (hydrophobic binding is favored by the presence of high salt, e.g., between about 0.1 M and 5.5 M, concentration) to exhaustively cleaned cotton thread or wetted thread (not exhaustively washed) has been demonstrated to yield a control reagent which will subsequently bind with the antibody function of the conjugate. Control thread prepared by mixing 1.76 mg IgG/100 mg thread (23 mg IgG/320 mL 0.5 $NaHCO_3$ buffer reagent) exhibited uniform color. That hydrophobic binding is operable by this adsorption approach is further corroborated by studies which demonstrate that the control thread loses its binding capacity when subjected to 20% dimethylformamide (DMFA). The loss of function is not attributed to protein denaturation, since covalently attached IgG retains its function when treated with DMFA.

EXAMPLE 3

Preparation of Polyester Control Thread

A polyester thread (50 mg) is incubated for 10-15 minutes in a mixed acid bath (5.0 mL) which is chilled to 4-5°C. The bath is composed of 40% $H_2SO_4$, 40% $HNO_3$ and 20% $H_2O$. Sulfuric acid concentration can be critical because lower concentrations, e.g., 30%, have resulted in poor functionality of the thread. Alternatively, concentrations above 40%, e.g., 50%, can lead to disintegration of the thread within 3-5 minutes. Higher temperature of the bath can also be deleterious to the integrity of the thread.

The reaction is stopped by dipping the thread into 50-100 mL of previously chilled $H_2O$. Caution should be maintained to insure the acid-treated thread is placed immediately into the $H_2O$ bath. Exposure to air permits the thread to adsorb moisture which initiates excessive heating due to the solvolysis of $H_2O$. An elevation in thread temperature at this point can cause the thread to disintegrate rapidly. The thread is made free of the acids with repeated washing with cold $H_2O$. When the pH of the water bath comes to neutrality, the thread is then soaked (two sequential rinses) in 5 mL of PBS, (20 mM $PO_4$, 30 mM NaCl, pH 7.0.

The thread is incubated with rabbit anti-(goat IgG) antibody (RAG) (0.1 mg/mL) in PBS at a thread to RAG ratio of 100:1 (w/w). Adsorption is carried out at room temperature for 24 hours with occasional stirring.

After the adsorption reaction, the thread is removed and immersed in 3.0 mL of a BSA-containing solution (5 mg/ml in PBS). This blocking reaction is held 24 hours at room temperature. The thread is then washed three times with 3.0 mL PBS, (20 mM $PO_4$, 30 mM NaCl, pH 7.4) and the thread can be stored in PBS (0.1 M $PO_4$, 100 mM NaCl, pH 7.4).

EXAMPLE 4

Preferred Methods of Coupling to Thread Element

A. Covalent coupling

In this method a reactive group such as hydrazide is attached to a thread which has been activated by an overnight treatment with periodic acid.

Cotton thread (1 g) was stirred gently in the dark with a freshly prepared solution of periodic acid (1.7 g in 140 mL of 0.7% acetic acid). After overnight or a longer reaction time, the thread was made free of the periodic acid with several washes of water. By spectrophotometric determinations, it was calculated that about 9-12% of the periodic acid is consumed during the reaction which is equivalent to 0.5 μmole/cm or 1 μmole/mg of the thread.

The periodate oxidized thread was gently stirred with adipic dihydrazide (1 g or greater dissolved as a 14 mg/mL solution in 0.7% acetic acid). After overnight incubation, the thread was copiously washed with water until the final wash was negative for adipic dihydrazide by the TNBS test [Anal. Biochem. 175:139-144 (1988)].

Rabbit anti-(goat IgG) antibody (RAG) (20 mg) was dialyzed overnight against 200 mL of 0.1 M acetate buffer containing 0.1 M NaCl, pH 5.5, and then against the same volume of the buffer for an additional four to five hours. The solution was centrifuged at 15,000 rpm for 10 minutes and the amount of protein was determined spectrophotometrically using a value for $A_{280}$ of 1.5 for a 1 mg/mL solution. The protein was treated with sodium metaperiodate (1.5 times the protein amount) in the dark at 4°C. After an overnight incubation, the reaction was arrested by 100 μL of 50% glycerol and the solution was dialyzed against 100 mL of the acetate buffer for two to three hours, the dialysis step was repeated two more times and the solution was centrifuged at 15,000 rpm for ten minutes. The protein in the supernatant was quantitated spectrophotometrically and the amount of carbonyl groups generated by the reaction were determined by the PAS-Schiff reaction using glycogen as a standard. In a typical procedure, an $A_{550}$ of 0.15-0.2 per mg of the protein or 16-22 μg glycogen equivalent per mg of the protein was found acceptable for its binding to the thread.

The adipidated thread (1 g) was stirred gently with 20 mg of the periodated RAG in a volume of 150 mL of the acetate buffer. After overnight or sometimes a longer reaction period, the thread was washed several times with 0.1 M $PO_4$ containing 0.15 M NaCl, pH 7.0 dried at room temperature for three to four hours, and stored at 4°C till further use.

B. Hydrophobic coupling

Replacement of adipic dihydrazide with phenylhydrazine in part A above leads to the formation of a thread which is exceedingly hydrophobic. The thread thus produced can be replaced as a binding matrix in the hydrophobic coupling mentioned in the earlier examples.

In a typical reaction, the peroxidized thread (226 mg) solution was stirred gently at room temperature in a phenyl hydrazine solution (253 mg dissolved in 100 mL of 0.2 M HCl). After 80 minutes, the thread was washed five times with 0.2 M HCl (100 mL each time), and finally with water to remove the acid, and then stored at 4°C until further use.

The present invention has been particularly described and exemplified above. Clearly, many other variations and modifications of the invention can be made without departing from the spirit and scope hereof.

## Claims

1. A test device for use in an immunoassay method for determining the presence of an analyte in a liquid test medium, in which method a liquid test sample or a liquid test mixture comprising a test sample is passed through a porous solid support having a test surface capable of immobilizing analyte or analyte bound by a specific binding partner thereof, and wherein analyte that becomes immobilized at said surface is determined by reaction with a reagent system to produce an optical response on said surface,

   characterized in that the test device further comprises a thread in liquid flow contact with and extending across said test surface, said thread being incorporated with a control reagent that reacts with one or more components of said reagent system to produce an optical response, preferably the production of color, on the thread independent of the presence of the analyte in the test medium.

2. The test device of claim 1 wherein the control reagent is analyte or an analog thereof, or a substance that reacts with less than all of the components of the reagent system to produce said optical response.

3. The test device of any of claims 1 and 2 wherein either (a) the reagent system comprises a labeled antibody reagent directed against the analyte, and wherein the control reagent is an antibody, or fragment thereof, directed against said labeled antibody reagent, or (b) the reagent system comprises an enzyme-labeled antibody reagent and a substrate/indicator composition which produces said optical response upon reaction with the enzyme comprised in the enzyme-labeled antibody reagent, and wherein the control reagent is said enzyme.

4. The test device of any of claims 1 to 3 wherein the test surface comprises an immobilized antibody reagent directed against the analyte, whereby upon passage of the test medium through the test surface of the device, the analyte becomes immobilized at such surface by becoming bound to said immobilized antibody reagent.

5. The test device of claim 4 wherein the antibody reagent is immobilized on a limited area of the exposed test surface and in a pattern that, cooperatively with the thread, provides a pattern of optical response indicative of a positive test result when the test medium contacted with the device contains a detectable amount of analyte, preferably said pattern being a thin band positioned perpendicular to the thread extended across the test surface, whereby a positive test result produces a "+" pattern of optical response and a negative test result produces only a "-" pattern of optical response due to the production of optical response only on the thread.

6. The test device of claim 4 wherein the antibody reagent is immobilized on substantially the entire exposed test surface, and wherein when the test medium can be applied to a limited area of such test surface and in a pattern that, cooperatively with the thread, provides a pattern of optical response indicative of a positive test result when the test medium contacted with the device contains a detectable amount of analyte, preferably said pattern being a cross comprising a thin band applied along the length of the thread and a thin band applied perpendicular to the thread extended across the test surface, whereby a positive test result produces a "+" pattern of optical response and a negative test result produces only a "-" pattern of optical response due to the production of optical response only on the thread.

7. The test device of any of claims 1 to 6 wherein the test surface is impervious to immune complexes formed between the analyte and an antibody reagent directed against the analyte, whereby upon passage of the test medium through the test surface of the device, the analyte becomes immobilized at such surface due to the inability of said immune complexes to pass therethrough.

8. The test device of any of claims 1 to 7 wherein the test medium can be applied to a limited area of the exposed test surface and in a pattern that provides, cooperatively with the thread, a pattern of optical response indicative of a positive test result when the test medium contacted with the device contains a detectable amount of analyte, preferably said pattern being a cross comprising a thin band applied along the length of the thread and a thin band applied perpendicular to the thread extended across the test surface, whereby a positive test result produces a " + " pattern of optical response and a negative test result produces only a "-" pattern of optical response due to the production of optical response only on the thread.

9. The test device of any of claims 1 to 8 wherein the porous solid support comprises a filter or membrane in liquid flow contact with an absorbent reservoir.

10. Use of the test device of any of claims 1 to 9 in an immunoassay method.

FIG. 1

FIG. 2

EP 0 451 686 A2

FIG. 3

FIG. 4

FIG. 5

12

FIG. 6

FIG. 6a

FIG. 6b